# EUROPEAN PATENT SPECIFICATION

(11) **EP 1 846 355 B1**
(45) Date of publication and mention of the grant of the patent: **16.12.2009**
(21) Application number: 06765423.6
(22) Date of filing: 17.01.2006
(51) Int. Cl.: C07C 69/52, C07C 69/587, C07C 67/14, A61K 31/21

(54) **FATTY ACID-BENZENEDIOL DERIVATIVES AND METHODS OF MAKING AND USING THEREOF**
FETTSÄURE-BENZENEDIOL-DERIVATE SOWIE HERSTELLUNGS- UND VERWENDUNGSVERFAHREN DAFÜR
DERIVES DE BENZENEDIOL D'ACIDES GRAS ET LEURS METHODES DE CONCEPTION ET D'UTILISATION

(30) Priority: 27.01.2005 US 647545 P
(43) Date of publication of application: 24.10.2007
(73) Proprietor: Ocean Nutrition Canada Limited, Dartmouth Nova Scotia B2Y 4T6 (CA)
(72) Inventor: BARROW, Colin, James, Halifax, NS B3H 1A8 (CA); KRALOVEC, Jaroslav, A., Halifax, NS B3S 1H4 (CA)
(74) Representative: Baldock, Sharon Claire
(86) International application number: PCT/IB2006/001229
(87) International publication number: WO 2006/117675

(56) References cited:
- WO-A-03/082233
- FR-A- 947 640
- FR-A- 1 175 829
- FR-A- 2 056 507
- GB-A- 1 533 086
- US-A- 3 969 383
- US-A- 4 036 773
- US-A- 4 526 779
- US-A- 6 127 417
- US-A1- 2003 236 202
- US-B1- 6 417 233
- PATENT ABSTRACTS OF JAPAN vol. 1995, no. 09, 31 October 1995 (1995-10-31) & JP 07 165575 A (NIPPON OIL & FATS CO LTD), 27 June 1995 (1995-06-27)
- PATENT ABSTRACTS OF JAPAN vol. 009, no. 005 (C-260), 10 January 1985 (1985-01-10) & JP 59 161308 A (SHISEIDO KK), 12 September 1984 (1984-09-12)
- PATENT ABSTRACTS OF JAPAN vol. 007, no. 273 (C-198), 6 December 1983 (1983-12-06) & JP 58 154507 A (SUNSTAR HAMIGAKI KK), 14 September 1983 (1983-09-14)
- HOPPER, RAGAN, HOOKS, LYNCH, MACDONALD: "Structure-activity relationships of Lysophosphatidic acid: conformationally restricted backbone mimetics" JOURNAL OF MEDICINAL CHEMISTRY, vol. 42, 1999, pages 963-970, XP002400294

## Description

### FIELD

The disclosed matter relates to compounds comprising fatty acids and benzenediol derivatives, including methods of making and using such compounds.

### BACKGROUND

Benzenediols are an important class of compounds with varied properties and uses. For example, one subclass ofbenzenediols is ubiquinol, a reduced form of Coenzyme Q. Coenzymes Q are also called ubiquinones, mitoquinones, or ubidecarerones, and they are lipophilic, water-insoluble substances involved in electron transport and energy production in mitochondria. The basic structure of coenzymes Q comprises a benzoquinone "head" and a terpinoid "tail." The "head" structure participates in the redox activity of the electron transport chain. The major difference among the various coenzymes Q is in the number of isoprenoid units (5-carbon structures) in the "tail." Coenzymes Q typically contain from 1 to 12 isoprenoid units in the "tail"; 10 isoprenoid units are common in animals such as mammals and man.

Coenzymes Q occur in the majority of aerobic organism, from bacteria to plants and animals. Two numbering systems exist for designating the number of isoprenoid units in the terpinoid "tail": coenzyme Qₙ and coenzyme Q(x), where n refers to the number of isoprenoid side chains and x refers to the number of carbons in the terpinoid "tail" and can be any multiple of five. Thus, coenzyme Q₁₀ (also termed CoQ₁₀) refers to a coenzyme Q having 10 isoprenoid units in the "tail." Since each isoprenoid unit has five carbons, CoQ₁₀ can also be designated coenzyme Q(50) or CoQ(50). The name CoQₙ can be used to generally refer to both the oxidized form and reduced form of the compound; alternatively, these specific forms can be individually designated CoQ_{nred} and CoQₙₒₓ. Chemically, CoQ₁₀ₙₒₓ is known as 2,3-dimethyoxy-5-methyl-6-decaprenyl-1,4-benzoquinone, and its structural formula is:

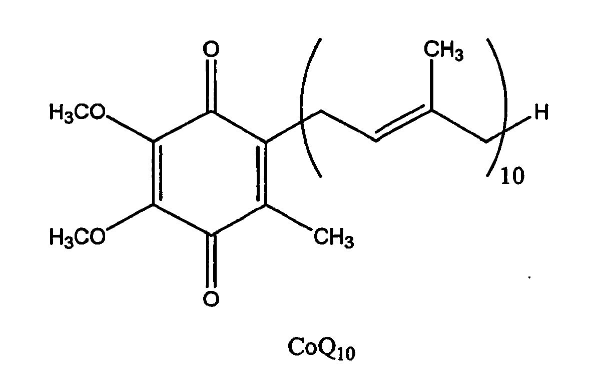

CoQ₁₀ is a model carrier of protons and electrons. It plays a vital role in the mitochondrial respiratory chain and oxidative phosphorylation. It was first isolated by researchers working at the Enzyme Institute of the University of Wisconsin (Crane, et al., BBA 25:220-1, 1975). Currently, Japanese Kaneka Corp. supplies 60 -70 % of CoQ₁₀ sold in the USA.

The oxidized form of CoQ₁₀ (CoQ₁₀ₒₓ) has anti-atherogenic properties. Deficiencies in CoQ₁₀ₒₓ are associated with higher incidence of heart failure and other cardiovascular problems. Although CoQ₁₀ plays an important role in the development of cardiovascular disease, there have been data that suggest that the coenzyme also plays an important role in the nervous system. For example, CoQ₁₀ is believed to have beneficial effects in the prevention and treatment of Parkinson's disease, mitochondrial myopathies, muscular dystrophy, etc.

Several attempts have been made to deliver benzendiol derivatives such as CoQ₁₀ to a subject. Selzer disclosed a liquid dietary CoQ₁₀ supplement based on vegetable oil-water emulsion. The absorption of CoQ₁₀ from this formulation was enhanced (U.S. Pat. No. 6,652,891 to Selzer et al.*).*

Herbamed developed a CoQ₁₀ formulation based on Emulsome technology that exhibits superior absorption. The product, called Ultrasome-CoQ₁₀, was tested on end-stage heart failure patients awaiting cardiac transplantation at the Rabin Medical Center and Sorasky Medical Center, both affiliated with Tel-Aviv University medical school (Berman M, Erman A, Ben-Gal T, Dvir D, Georghiou GP, Stamler A, Vered Y, Vidne BA, Aravot D. Coenzyme Q10 in patients with end-stage heart failure awaiting cardiac transplantation: a randomized, placebo-controlled study. Clin Cardiol 2004, 27:295-9). The product was found to be three times more bioavailable than generic CoQ₁₀. In the double blind trial, 32 patients awaiting heart transplantation received either 60 mg of the product or placebo for three months. The Ultrasome group showed significant improvement in a six-minute walk test and a decrease in dyspnea (New York Heart Association classification), nocturia, and fatigue, compared to the placebo.

Natural Health Sciences together with General Nutrition Centers developed a blend of Pycnogenol, a French maritime pine bark extract, and CoQ₁₀ called PycnoQ10. Joint research executed at Showa Medical University, Tokyo, and State University of New York suggested that the combination protected 53% of blood lipids from oxidation compared to 30% when the ingredients were used separately. The product protected blood vessel integrity, blood lipid values, circulation, blood pressure, and platelet function. The activity is believed to be derived from the synergy of antioxidant properties.

Guarnieri et al disclose stable derivatives of ubiquinole that are useful in the treatment of oxidative stress (U.S. Pat. No. 6,127,417).

Horroobin describes a physical mixture of CoQ₁₀ and eicosapentaenoic acid (EPA) (Int'l. Pub. No. WO 02/096408 A1). Sears, *et .al.,* describes a composition made of CoQ₁₀ and polyunsaturated fatty acids (PUFA) such as docosahexaenoic acid (DHA), EPA, or linolenic acid, which is intended for the prevention and/or treatment of mitochondriopathies (U.S. Pat. No. 6,417,233). Formation of the ester between PUFA and CoQ₁₀ is not disclosed. U.S. Pat. Nos. 6,300,377 and 6,441,050 to Chopra disclose a combination of CoQ₁₀ with a polysorbate surfactant, which can also be mixed with other active materials such as omega-3 fatty acids.

In light of the numerous health benefits associated with benzendiol derivatives such as CoQ₁₀, what is needed in the art are new compounds and compositions that can be used to supply such benzendiol derivatives to subjects. Further, what are also needed are new methods of preparing and using such compounds and compositions. The compounds, compositions, and methods disclosed herein meet these needs and other needs.

### SUMMARY

In accordance with the purposes of the disclosed materials, compounds, compositions, articles, and methods, as embodied and broadly described herein, the disclosed subject matter, in one aspect, relates to compounds and compositions and methods for preparing and using such compounds and compositions. In another aspect, the disclosed subject matter relates to compounds comprising Formula IV: 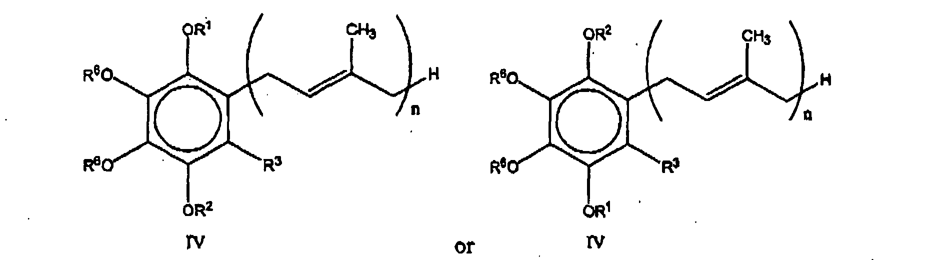 wherein R¹ is an omega-3 unsaturated fatty acid residue; R³ is hydrogen or an omega-3 unsaturated fatty acid residue; R³ and each R⁶ is methyl and n is 14. In a further aspect, disclosed herein are nutritional supplements, food stuffs, and pharmaceutical formulations comprising such compounds. In still another aspect, the disclosed subject matter relates to methods of preparing such compounds and compositions. Still further, the disclosed subject matter relates to microcapsules containing, such compounds and compositions and to methods. of preparing the microcapsules. In yet another aspect, the disclosed subject matter relates to methods of using the described compounds and compositions.

Additional advantages will be set forth in part in the description that follows, and in part will be obvious from the description, or may be learned by practice of the aspects described below. The advantages described below will be realized and attained by means of the elements and combinations particularly pointed out in the appended claims. It is to be understood that both the foregoing general description and the following detailed description are exemplary and explanatory only and are not restrictive.

### BRIEF DESCRIPTION OF FIGURES

The accompanying Figures, which are incorporated in and constitute a part of this specification, illustrate several aspects described below.
Figure 1 is flow injection analysis of a CoQ₁₀ fatty acid conjugate prepared according to Examples 4-6.
Figure 2 is an ESI mass spectrum obtained from total ion current resulting from flow injection analysis of a CoQ₁₀ fatty acid conjugate that was prepared according to Examples 4-6.
Figure 3 is a fragmentation spectrum resulting from selected precursor anions (8827,1 1167.0, and 1193.0) resulting from MS of CoQ₁₀ fatty acid conjugate that was prepared according to Examples 4-6.

### DETAILED DESCRIPTION

The materials, compounds, compositions, articles, and methods described herein may be understood more readily by reference to the following detailed description of specific aspects of the disclosed subject, matter and the Examples included therein and to the Figures.

Before the present materials, compounds, compositions, articles, and methods are disclosed and described, it is to be understood that the aspects descaled below are not limited to specific synthetic methods or specific reagents, as such may, of course, vary. It is also to be understood that the terminology used herein is for the purpose of describing particular aspects only and is not intended to be limiting.

### General Definitions

In this specification and in the claims that follow, reference will be made to a number of terms, which shall be defined to have the following meanings:
Throughout the description and claims of this specification the word "comprise" and other forms of the word, such as "comprising" and "comprises," means including but not limited to, and is not intended to exclude, for example, other additives, components, integers, or steps.
As used in the description and the appended claims, the singular forms "a," "an," and "the" include plural referents unless the context clearly dictates otherwise. Thus, for example, reference to "a compound" includes mixtures of two or more such compounds, reference to "an unsaturated fatty acid" includes mixtures of two or more such unsaturated fatty acids, reference to "the microcapsule" includes mixtures of two or more such microcapsules.

"Optional" or "optionally" means that the subsequently described event or circumstance can or cannot occur, and that the description includes instances where the event or circumstance occurs and instances where it does not

References in the specification and concluding claims to parts by weight of a particular element or component in a composition denotes the weight relationship between the element or component and any other elements or components in the composition or article for which a part by weight is expressed. Thus, in a compounds containing 2 parts by weight of component X and 5 parts by weight component Y, X and Y are present at a weight ratio of 2:5, and are present if such ratio regardless of whether additional components are contained in the compound.

A weight percent of a component, unless specifically stated to the contrary, is based on the total weight of the formulation or composition in which the component is included.

As used herein, the term "substituted" is contemplated to include all permissible substituents of organic compounds. In a broad aspect, the permissible substituents include acyclic and cyclic, branched and unbranched, carbocyclic and heterocyclic, and aromatic and nonaromatic substituents of organic compounds. Illustrative substituents include, for example, those described below. The permissible substituents can be one or more and the same or different for appropriate organic compounds. For purposes of this disclosure, the heteroatoms, such as nitrogen or oxygen, can have hydrogen substituents and/or any permissible substituents of organic compounds described herein which satisfy the valencies of the heteroatoms. This disclosure is not intended to be limited in any manner by the permissible substituents of organic compounds. Also, the terms "substitution" or "substituted with" include the implicit proviso that such substitution is in accordance with permitted valence of the substituted atom and the substituent, and that the substitution results in a stable compound, *e.g.*, a compound that does not spontaneously undergo transformation such as by rearrangement, cyclization, or elimination.

The term "alkyl" as used herein is a branched or unbranched saturated hydrocarbon group of 1 to 40 carbon atoms, such as methyl, ethyl, n-propyl, i-propyl, n-butyl, i-butyl, s-butyl, t-butyl, pentyl, hexyl, heptyl, octyl, nonyl, decyl, dodecyl, tetradecyl, hexadecyl, eicosy and tetracosyl. The alkyl group can also be substituted or unsubstituted. The alkyl group can be substituted with one or more groups including alkyl, halogenated alkyl, alkoxy, alkenyl, alkynyl, acryl, aldehyde, amino, carboxylic acid, ester, ether, halide, hydroxy, ketone, nitro, silyl, sulfo-oxo, or thiol, as described below.

The term "alkoxy" or "alkoxide" as used herein is an alkyl group bound through a single, terminal ether linkage; that is, an "alkoxy" group may be defined as -OA where A is alkyl as defined above.

The term "alkenyl" as used herein is a hydrocarbon group of from 2 to 40 carbon atoms with a structural formula containing at least one carbon-carbon double bond. Asymmetric structures such as (AB)C=C(DE) are intended to include both the E and Z isomers *(cis* and *trans).* This may be presumed in structural formulae herein wherein an asymmetric alkene is present, or it may be explicitly indicated by the bond symbol C=C. The alkenyl group can be unsubstituted or substituted with one or more groups including, alkyl, halogenated alkyl, alkoxy, alkenyl, alkenyl, aryl, aldehyde, amino, carboxylic acid, ester, ether, halide, hydroxy, ketone, nitro, silyl, sulfo-oxo, or thiol, as described below.

The term "alkynyl" as used herein is a hydrocarbon group of 2 to 40 carbon atoms with a structural formula containing at least one carbon-carbon triple bond. The alkynyl group can be unsubstituted or substituted with one or more groups including alkyl, halogenated alkyl, alkoxy, alkenyl, alkynyl, aryl, aldehyde, amino, carboxylic acid, ester, ether, halide, hydroxy, ketone, nitro, silyl, sulfo-oxo, or thiol, as described below.

The term "aryl" as used herein is a group that contains any carbon-based aromatic group including, but not limited to, benzene, naphthalene, phenyl, biphenyl, and phenoxybenzene. The term "aryl" also includes "heteroaryl," which is defined as a group that contains an aromatic group that has at least one heteroatom incorporated within the ring of the aromatic group. Examples of heteroatoms include nitrogen, oxygen, sulfur, and phosphorus. The aryl group can be substituted or unsubstituted. The aryl group can be substituted with one or more groups including akyl, halogenated alkyl, alkoxy, alkenyl, alkynyl, aryl, aldehyde, amino, carboxylic acid, ester, ether, halide, hydroxy, ketone, nitro, silyl, sulfo-oxo, or thiol as described herein.

Unless stated to the contrary, a formula with chemical bonds shown only as solid lines and not as wedges or dashed lines contemplates each possible isomer, *e.g.*, each enantiomer and diastereomer, and a mixture of isomers, such as a racemic or scalemic mixtures.

As used herein, by a "subject" is meant an individual. Thus, the "subject" can include domesticated animals (*e.g.*, cats, dogs), livestock (*e.g.*, cattle, horses, pigs, sheep, goats), laboratory animals (*e.g.*, mouse, rabbit, rat, guinea pig), and birds. "Subject" can also include a mammal, such as a primate or a human.

The term "increase," or other forms of increase, such as "increasing," refers to an increase in an event or characteristic above basal levels, *e.g.*, as compared to a control. The terms "reduces" or "lowers," or other forms the words, such as "reducing," "reduction," or "lowering," refers to a decrease in an event or characteristic below basal levels, *e.g.*, as compared to a control. By "control" is meant either a subject, organ, tissue, or cell lacking a disease or injury, or a subject, organ, tissue, or cell in the absence of a particular variable such as a therapeutic agent. A subject, organ, tissue, or cell in the absence of a therapeutic agent can be the same subject, organ, tissue, or cell before or after treatment with a therapeutic agent or can be a different subject, organ, tissue, or cell in the absence of the therapeutic agent. Comparison to a control can include a comparison to a known control level or value known in the art. Thus, basal levels are normal *in vivo* or *in vitro* levels prior to, or in the absence of, the addition of an agent (*e.g.*, a therapeutic agent) or another molecule.

By "prevent" or other forms of prevent, such as "preventing" or "prevention," is meant to stop a particular event or characteristic, to stabilize or delay the development or progression of a particular event or characteristic, or to minimize the chances that a particular event or characteristic will occur. Prevention does not require comparison to a control as it is typically more absolute than, for example, reduce or lower. As used herein, something could be reduced or lowered but not prevented, but something that is reduced or lowered could also be prevented. Likewise, something could be prevented but not reduced or lowered, but something that is prevented could also be reduced or lowered. It is understood that where reduce, lowered, or prevent are used, unless specifically indicated otherwise, the use of the other two words is also expressly disclosed. Thus, if lowering cholesterol levels is disclosed, then reducing and preventing cholesterol levels are also disclosed.

By "treat" or other forms of treat, such as "treated" or treatment," is meant to administer a composition disclosed herein or to perform a method disclosed herein in order to reduce or prevent a particular characteristic or event (*e.g.*, mitochondrial disease).

Reference will now be made in detail to specific aspects of the disclosed materials, compounds, compositions, articles, and methods, examples of which are illustrated in the accompanying Examples and Figures.

### Materials

Disclosed herein are materials, compounds, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a compound is disclosed and a number of modifications that can be made to a number of components or residues of the compound are discussed, each and every combination and permutation that are possible are specifically contemplated unless specifically indicated to the contrary. Thus, if a class of components or residues A, B, and C are disclosed as well as a class of components or residues D, E, and F, and an example of a combination compound A-D is disclosed, then even if each is not individually recited, each is individually and collectively contemplated. Thus, in this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-B are specifically contemplated and should be considered disclosed from disclosure of A, B, and C; D, E, and F; and the example combination A-D. This concept applies to all aspects of this disclosure including, but not limited to, steps in methods of making and using the disclosed compositions. Thus, if there are a variety of additional steps that can be performed it is understood that each of these additional steps can be performed with any specific aspect or combination of aspects of the disclosed methods, and that each such combinations is specifically contemplated and should be considered disclosed.

Certain materials, compounds, compositions, and components disclosed herein can be obtained commercially or can be readily synthesized using techniques generally known to those of skill in the art. For example, the starting materials and reagents used in preparing the disclosed compounds and compositions are either available from commercial suppliers such as Aldrich Chemical Co., (Milwaukee, Wis.), Acros Oxganics (Morris Plains, NJ.), Fisher Scientific (Pittsburgh, Pa.), or Stigma (St Louis. Mo.) or are prepared by methods known to those skilled in the art following, procedures set forth in references such as Fieser and Fieser's Reagents for Organic Synthesis, Volumes 1-17. (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991); March's Advanced Organic Chemistry, (John Wiley and Sons, 4th Edition); and Larock's Comprehensive Organic Transformations (VCH Publishers Inc., 1989).

### Quinone derivative-fatty acid compounds

For example, the fatty acids and residues thereof can comprise from 10 to 40, from 12 to 38, from 14 to 36, from 16 to 34, from 18 to 32, or from 20 to 30 carbon atoms.

Specific examples of suitable fish oils include, but are not limited to, Atlantic fish oils, Pacific fish oils, Mediterranean fish oils, light pressed fish oil, alkaline treated fish oil, heat treated fish oil, light and heavy brown fish oil, tuna oil, sea bass oil, halibut oil, spearfish oil, barracuda oil, cod oil, menhaden oil, sardine oil, anchovy oil, capelin oil, Atlantic cod oil, Atlantic herring oil, Atlantic mackerel oil, Atlantic menhaden oil, salmonids oil and shark oil

### Omega-3 fatty acids

Omega-3 fatty acids are certain unsaturated fatty acids that are particularly useful in the compounds and methods disclosed herein. Omega-3 fatty acids not only exhibit proven effects on lowering serum triglyceride levels, but they have strong connection to diabetes. For instance, docosahexaenoic acid (DHA) also has a strong insulin permeability enhancement effect, and it is viewed as a potential absorption enhancer for intestinal delivery of insulin (Onuki, et al., Int J Pharm 198:147-56, 2000). DHA intake can prevent certain biochemical processes that originate from insulin deficiency (Ovide-Bordeaux and Grynberg, Am J Physiol Regul Integr Comp Physiol 286:R519-27, 2003) and both DHA and EPA (eicosapentaenoic acid) can significantly increase fasting insulin levels (Mori, et al., Am J Clin Nutr 71:1085-94, 2000).

An omega 3 fatty acid is an unsaturated fatty acid that contains as its terminus CH₃-CH₂-CH=CH.-. Specific examples of omega-3 fatty acids that are suitable for use herein include linolenic acid (18:3ω3), octadecatetraenoic acid (18:4ω3), eicosapentaenoic acid (20:5ω3) (EPA), docosahexaenoic acid (22:6ω3) (DHA), docosapentaenoic acid (22:6ω3) (DPA), derivatives thereof, and combinations thereof.

In still other examples, the unsaturated fatty acids or residues thereof can be derived from a compound comprising Formula II: 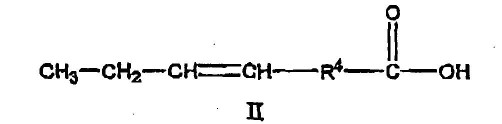 wherein R⁴ can be a C₃-C₄₀ alkyl or alkenyl group comprising at least one double bond. In a further example, R⁴ can be a C₅-C₃₈, C₆-C₃₆, C₈-C₃₄, C₁₀-C₃₂, C₁₂-C₃₀, C₁₄-C₂₈, C₁₆-C₂₆ or C₁₈-C₂₄ alkenyl group. In yet another example, the alkenyl group of R⁴ can have from 2 to 6, from 3 to 6, from 4 to 6, or from 5 to 6 double bonds. Still further, the alkenyl group of R^{.4} can have from 1, 2, 3, 4, 5, or 6 double bonds, where any of the stated values can form an upper or lower endpoint when appropriate.

### Exemplary unsaturated fatty acids

In one aspect, the unsaturated fatty acid residue can be derived from eicosapentaenoic acid 20:5ω3 (EPA), docosahexaemoic acid 22:6ω3 (DRA), docosapentaenoic acid 22:5ω3 (DPA), and any combination thereof.

### Permutations of R¹ and R²

In one aspect, the disclosed compounds comprising Formula IV can have R¹ being any of the omega 3 unsaturated fatty acid residues disclosed above. Further, in another aspect, the disclosed compounds can have R¹ being any of the unsaturated fatty acid residues disclosed above and R² can be H. In yet another aspect, the disclosed compounds can have R¹ and R² each being any of the unsatured fatty acid residues disclosed above. For example, R¹ and R² can be the same unsaturated fatty acid residue or, in another example, R¹ and R² can be different unsaturated fatty acid residues. In a further aspect R¹ can be any of the unsaturated fatty acid residues disclosed above and R² can be any of the saturated fatty acid residues disclosed above.

In some particular example, R¹ and R² can be unsaturated fatty acid residues derived from fish oil. In other examples, R¹ and R² can be unsaturated fatty acid residues comprising at least 20 carbon atoms. In yet other examples, R¹ and R² can be unsaturated fatty acid residues comprising at least one pair of methylene interrupted unsaturated bonds. In still other examples, R¹ and R² can be unsaturated fatty acid residues derived from an omega-3 fatty acid.

In other specific examples of the disclosed compounds, R¹ and R² can be unsaturated fatty acid residues derived from a compound composing Formula II:

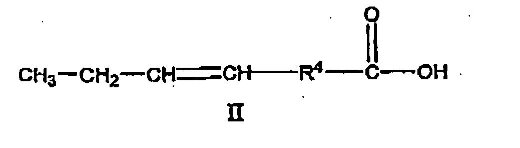

In further examples, R¹ and R² can be unsaturated fatty acid residues derived from eicosapentaenoic acid 20:5ω3 (EPA), docosahexaenoic acid 22:6ω3 (DHA), docosapentaenoic acid 22:5ω3 (DPA), or any combination thereof.

### Exemplary Compounds

Some additional examples of compounds disclosed and described herein can include, compounds comprising the following Formula IV: 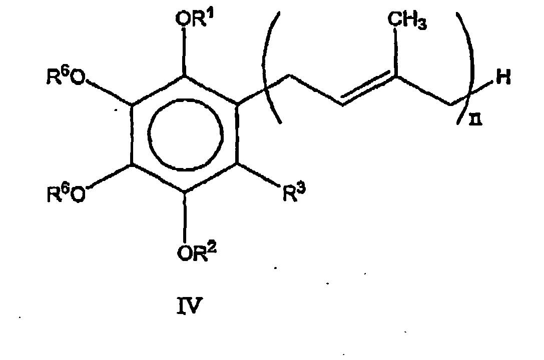 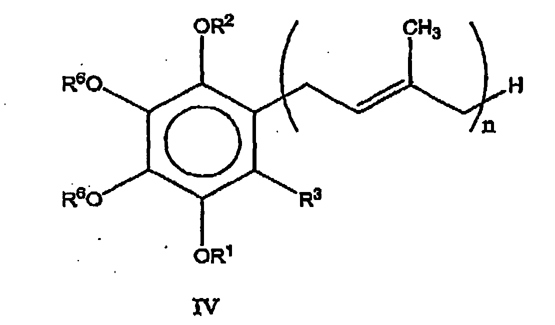 or wherein R¹, R², R³, R⁶ and n are as previously described.

In still another example, either R¹ or R² or both R¹ and R² can be derived atom eicosapentaenoic acid 20:5ω3 (EPA), docosahexaenoic acid 22:6ω3 (DHA), docosapentaenoic acid 22:5ω3 (DPA), or any combinations thereof

### Additional properties

The disclosed compounds can be, in one aspect, bioavailable. "Bioavailable" means that a compound is in a form that allows for it, or a portion of the amount administered, to be absorbed by, incorporated into, or otherwise physiologically available to a subject or patient to whom it is administered.

Further, chemical coupling benzenediol derivatives with fatty acids, as disclosed herein, can yield syrup-like compounds that are more palatable than a corresponding heterogeneous solid/liquid mixture.

### Methods of Making

Also disclosed herein are methods for preparing the disclosed compounds and related compounds. In one aspect, the compounds can be prepared by reacting a compound comprising Formula III; 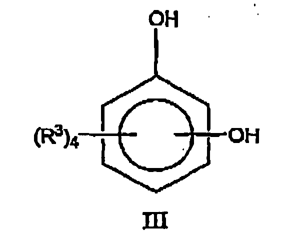 wherein R³ is, independently, H, OH, alkyl, alkoxyl, alkenyl, or alkynyl with one or more unsaturated fatty acids or a derivative thereof.

In one aspect, the compound represented by Formula IIIcan have can have the substituents OH in the para-, meta , or ortho-positions *(e.g.,* derivatives of hydroquinone (1,4-benzenediol), resorcinol (1,3-benzenedion, and catechol (1,2-benzendiol), respectively.

In another aspect, of the compound represented by Formula III, R³ can be, independently. H, OH, alkyl, alkoxide, alkenyl, or alkynyl, as defined above. For example, at least one R³ substituent can be a methyl, ethyl, or propyl. In another example, at least one R³ substituent can be a methoxide, ethoxide, or propoxide. In yet another example, at least one R³ substituent can be a alkenyl group having the formula - [CH₂CH=C(CH₃)CH₂-]ₙ-H, where n is an integer of from 1 to 12. In still another example, one R³ substituent in Formula III can be -[CH₂CH=C(CH₃)CH₂-]ₙ-H, where n is an integer of from 1 to 12, one R³ substituent can be methyl, and two R³ substituents can be methoxy.

In one specific example, the compound represented by Formula III can comprise Fomula VI; 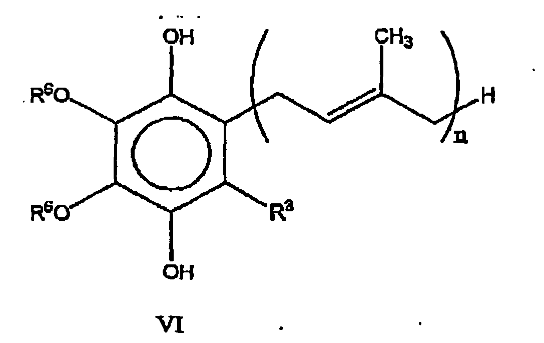 Herein R³ and each R⁶ can be an alkyl group and n can be from 1 to 12, for example, the compound represented by Formula VI can be CoQ₁₋₁₂. In one specific example, R³ and each R⁶ can be methyl and n is 10. This compound is the reduced form of CoQ₁₀, which is referred to herein as CoQ_{10red}. CoQ_{10red} can be produced from CoQ₁₀ by reacting CoQ₁₀ with a reducing agent such as, for example, NaBH₄ or hydrogenation with Zn and AcOH-Various techniques axe described herein for producing CoQ_{10red}.

### Unsaturated fatty acids

In a further example, the unsatured fatty acid or derivative thereof can be an omega-3 fatty acid. In still another example, the unsaturated fatty acid or derivative thereof can comprise the Formula II: 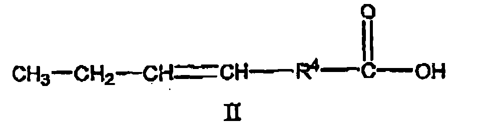 wherein R⁴ can be a C₃-C₄₀ alkyl or alkenyl group comprising at least one double bond. The substituent R⁴ can have from 2 to 6 double bonds.

In other specific examples, the unsaturated fatty acid or derivative thereof can comprise eicosapentaenoic acid 20:5ω3 EPA), docosahexaenoic acid 22:6ω3 (DHA), docosapentaenoic acid 22:5ω3 (DPA), or any combination thereof.

In one particular aspect, the compound represented by Formula VI, wherein R³ and each R⁶ is a methyl group and n is 10 is reacted with an unsaturated fatty acid or derivative thereof derived from fish oil.

### Concentration

In the methods disclosed herein, the reaction with the compounds represented by Formula III or VI and one or more unsaturated fatty acid or derivatives thereof can take place under various conditions. For example, the reaction can take place neat. In another aspect, the reaction can take place in any solvent. For example, the reaction can take place in an aqueous solvent such as water, aqueous hexane, aqueous ethanol, aqueous methanol, and aqueous propanol. The reaction can also take place in non-aqueous solvents, such as DMSO DMF, THF, benzene, toluene, hexane, pentane, dichloromethane, acetome and pyridine. In another example, the reaction can take place in a diphasic system containing an aqueous phase and an organic phase. In these systems, suitable organic phases can contain, for example, butanol, pentane, cyclopentane, hexane, cyclohexane, heptane, benzene, toluene, carbon tetrachloride, chloroform, methylene chloride, dichloroethane, ethyl acetate, ether, MEK, octane, diisopropyl ether, tn and tetrachlorethane. The amount of solvent used and the concentration of the compound of Formula III or VI and/or unsaturated fatty acid or derivative thereof will depend on the particular compound being prepared, the type ofbenzendiol derivative of Formula III or VI, the type of unsaturated fatty acid or derivative thereof, the type of solvent and preference

### Temperature

The compound of Formula III or VI can be reacted with the unsaturated fatty acid or derivative thereof at any temperature sufficient to form a bond between one or more hydroxyl substituents on the aromatic ring and the unsaturated fatty acid or derivative thereof. Typically, the reaction can take place at an elevated temperature. The precise elevated temperature can depend on the particular fatty acid being used, the particular benzenediol derivative of Formula III or VI being used, the solvent, the amount or concentration of the reagents, preference, and the like. Suitable temperatures at which the benzenediol derivative of Formula III or VI can be reacted with the fatty acid include from 20 to 200°C, from 50 to 220°C, from 70 to 240°C, from 90 to 260°C, or from 110 to 280°C. In other examples, the temperature of the reaction can be at 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, or 300°C, where any of the stated values can form an upper or lower endpoint when appropriate.

### Specific Examples

In one example, Scheme 1 illustrates the direct acylation of the reduced form of CoQ₁₀ (*i.e*., Formula VI with R³ and each R⁶ being methyl and n =10, with a DHA derivative, where X is a leaving group. Coenzyme Q₁₀ (CoQ₁₀) is available generically from numerous manufacturers. Branded products include Lynae CoQ₁₀ (Boscogen, Irvine CA), Natures Blend Coenzyme Q₁₀ (National Vitamin Company, Porterville, CA) and Ultra CoQ₁₀ (Twinlab, Hauppauge, NY).

The scheme is written only for acylation of one of the two hydroxy groups but it is contemplated that either or both groups can be acylated and the ratio would depend on the reaction conditions.

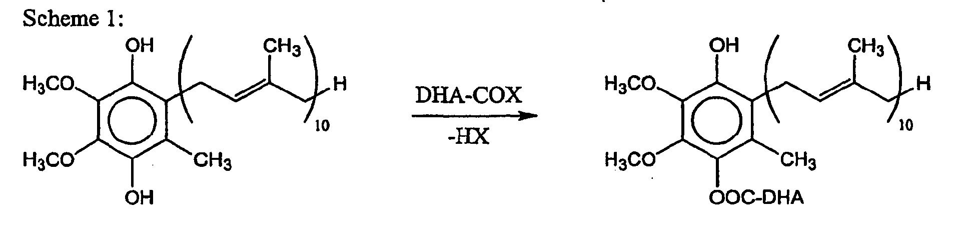

Also, disclosed herein are compounds prepared by the methods disclosed herein.

### Supplements

Also disclosed herein are nutritional supplements. A nutritional supplement is any compound or composition that can be administered to or taken by a subject to provide, supply, or increase a nutrient(s) (*e.g*., vitamin, mineral, essential trace element, amino acid, peptide, nucleic acid, oligonucleotide, lipid, cholesterol, steroid, carbohydrate ). In one aspect, disclosed herein are nutritional supplements comprising any of the compounds disclosed herein. For example, a nutritional supplement can comprise any compound comprising Formula IV. The fatty acid residues of these formulas can be any fatty acid as disclosed herein.

The nutritional supplement can comprise any amount of the compounds disclosed, herein, but will typically contain an amount determined to supply a subject with a desired dose of a benzenediol derivative (*e.g*, CoQ₁₀) and/or fatty acids. The exact amount of compound required in the nutritional supplement will very from subject to subject, depending on the species, age, weight and general condition of the subject, the severity of the dietary deficiency being treated, the particular mode of administration, and the like. Thus, it is not possible to specify an exact amount for every nutritional supplement. However, an appropriate amount can be determined by one of ordinary skill in the art using only routine experimentation given the teachings herein. In one specific example, a nutritional supplement can comprise from 0.05 to 20%, from 1 to 7.5%, or from 3 to 5% by weight of the compound. In another example, the nutritional supplement can comprise from 0.05, 0.10. 0.15, 0.20, 025, 0.30, 0.35, 0.40, 0.45, 0.50, 0.55, 0.60, 0.65, 0.70, 0.75, 0.80, 0.85, 0.90, 0.95, 1.0, 1.5, 2.0, 225, 3.0, 3.5, 4.0, 4.5, 5.0, 5.5, 6.0, 6.5, 7.0, 7.5, 8.0, 8.5, 9.0, 9.5,10, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0,19.5, or 20.0% by weight of the compound, where any of the stated values can form an upper or lower endpoint when appropriate. In another aspect, when the nutritional supplement, the supplement can be composed of up to 100% of the supplement.

The nutritional supplement can also comprise other nutirient(s) such as vitamins trace elements and minerals. Further, the nutritional supplement can comprise other components such as preservatives, antimicrobials, anti-oxidants, chelating agents, thickeners, flavorings, diluents, emulsifiers, dispersing aids, and/or binders.

The nitritional supplements are generally taken orally and can be in any form suitable for oral administration. For example, a nutritional supplement can typically be in a tablet, gel-cap, capsule, liquid, sachets, or syrup form.

### Pharmaceutical formulation

Also, disclosed herein are pharmaceutical formulations. In one aspect, a pharmaceutical formulation can comprise any of the compounds disclosed herein with a pharmaceutically acceptable carrier. For example, a pharmaceutical formulation can comprise a compound comprising Formula IV and a pharmaceutically acceptable carrier. The disclosed pharmaceutical formulations can be used therapeutically or prophylactically.

By "pharmaceutically acceptable" is meant a material that is not biologically or otherwise undesirable, *i.e*., the material may be administered to a subject without causing any undesirable biological effects or interacting in a deleterious manner with any of the other components of the pharmaceutical formulation in which it is contained. The carrier would naturally be selected to minimize any degradation of the active ingredient and to minimize any adverse side effects in the subject, as would be well known to one of skill in the art.

Pharmaceutical carriers are known to those skilled in the art. These most typically would be standard carriers for administration of drugs to humans, including solutions such as sterile water, saline, and buffered solutions at physiological pH. Suitable carriers and their formulations are described in Remington: The Science and Practice of Pharmacy (19th ed.) Gennaro, ed., Mack Publishing Company, Easton, PA, 1995.

Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution is preferably from 5 to 8, and more preferably from 7 to 7.5. Further carriers include sustained release preparations such as semipermeable matrices of solid hydrophobic polymers containing the disclosed compounds, which matrices are in the form of shaped articles, *e.g*., films, liposomes, microparticles, or microcapsules. It will be apparent to those persons skilled in the art that certain carriers can be more preferable depending upon, for instance, the route of administration and concentration of composition being administered. Other compounds can be administered according to standard procedures used by those skilled in the art.

Pharmaceutical formulations can include additional carriers, as well as thickeners, diluents, buffers, preservatives, surface active agents and the like in addition to the compounds disclosed herein. Pharmaceutical formulations can also include one or more additional active ingredients such as antimicrobial agents, antiinflammatory agents, and anesthetics.

The pharmaceutical formulation can be administered in a number of ways depending on whether local or systemic treatment is desired, and on the area to be treated. Administration may be topically (including ophthalmically, vaginally, rectally, intranasally), orally, by inhalation, or parenterally, for example by intravenous drip, subcutaneous, intraperitoneal or intramuscular injection. The disclosed compounds can be administered orally, intravenously, intraperitoneally, intramuscularly, subcutaneously, intracavity, or transdermally.

Pharmaceutical formulations for oral administration include, but are not limited to, powders or granules, suspensions or solutions in water or non-aqueous media, capsules, sachets, or tablets. Thickeners, flavorings, diluents, emulsifiers, dispersing aids, anti-oxidants, or binders may be desirable.

Pharmaceutical formulations for parenteral administration include sterile aqueous or non-aqueous solutions, suspensions, and emulsions. Examples of non-aqueous solvents are propylene glycol, polyethylene glycol, vegetable oils such as olive oil, fish oils, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases

Pharmaceutical formulations for topical administration may include ointments, lotions, creams, gels, drops, suppositories, sprays, liquids and powders. Conventional pharmaceutical carriers, aqueous, powder or oily bases and thickeners may be necessary or desirable.

Some of the formulations can potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

### Delivery Devices

Any of the compounds described herein can be incorporated into a delivery device. Examples of delivery devices include, but are not limited to, microcapsules, microspheres, nanospheres or nanoparticles, liposomes, noisome, nanoerythrosome, solid-liquid nanoparticles, gels, gel capsules, tablets, lotions, creams, sprays, emulsions, Other examples of delivery devices that are suitable for non-oral administration include pulmospheres. Examples of particular delivery devices useful herein are described below.

The disclosed compounds can be incorporated into liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The disclosed compositions in liposome form can contain, in addition to a compound disclosed herein, stabilizers, preservatives and excipients. Examples of suitable lipids are the phospholipids and the phosphatidyl cholines (lecithins), both natural and synthetic. Methods of forming liposomes are known in the art. *See, e.g.,* Prescott, Ed., Methods in Cell Biology, Volume XIV, Academic Press, New York, p. 33 et seq., 1976,

In other examples, the liposomes can be cationic liposomes (*e.g*., DOTMA, DOPE, DC cholesterol) or anionic liposomes. Liposomes can further comprise proteins to facilitate targeting a particular cell, if desired. Administration of a composition comprising a compound and a cationic liposome can be administered to the blood afferent to a target organ or inhaled into the respiratory tract to target cells of the respiratory tract. Regarding liposomes, *see e.g.,* Brigham, et al., Am J Resp Cell Mol Biol 1:95-100, 1989; Felgner, et al., Proc Natl Acad Sci USA 84:7413-7, 1987; and U.S. Pat. No. 4,897,355.

As one example, delivery can be via a liposome using commercially available liposome preparations such as LIPOFECTIN, LIPOFECTAMINE (GIBCO-BRL, Inc., Gaithersburg, MD), SUPERFECT (Qiagen, Inc. Hilden, Germany) and TRANSFECTAM (Promega Biotec, Inc., Madison, WI), as well as other liposomes developed according to procedures standard in the art. Liposomes where the diffusion of the compound or delivery of the compound from the liposome is designed for a specific rate or dosage can also be used.

As described herein, niosomes are delivery devices that can be used to deliver the compositions disclosed herein. Noisomes are multilamellar or unilamellar vesicles involving non-ionic surfactants. An aqueous solution of solute is enclosed by a bilayer resulting from the organization of surfactant macromolecules. Similar to liposomes, noisomes are used in targeted delivery of, for example, anticancer drugs, including methotrexate, doxorubicin, and immunoadjuvants. They are generally understood to be different from transferosomes, vesicles prepared from amphiphilic carbohydrate and amino group containing polymers, *e.g*., chitosan.

As described herein, nanoerythrosomes are delivery devices that can be used to deliver the compositions disclosed herein. Nanoerythrosomes are nano-vesicles made of red blood cells via dialysis through filters of defined pore size. These vesicles can be loaded with a diverse array of biologically active molecules, including proteins and the compositions disclosed herein. They generally serve as ideal carriers for antineoplastic agents like bleomycin, actinomycin D, but can be used for steroids, or other lipids.

Artificial red blood cells, as described herein, are further delivery devices that can be used to deliver the compositions disclosed herein. Artificial red blood cells can be generated by interfacial polymerization and complex emulsion methods. Generally, the "cell" wall is made of polyphtaloyl L-lysine polymer/polystyrene and the core is made of a hemoglobin solution from sheep hemolysate. Hemoglobin loaded microspheres typically have particle sizes of from about 1 to about 10 mm. Their size, flexibility, and oxygen carrying capacity is similar to red blood cells.

Solid-lipid nanoparticles, as described herein, are other delivery devices that can be used to deliver the compositions disclosed herein. Solid-lipid nanoparticles are nanoparticles; which are dispersed in an aqueous surfactant solution. They are comprised of a solid hydrophobic core having a monolayer of a phospholipid coating and are usually prepared by high-pressure homogenization techniques. Immunomodulating complexes (ISCOMS) are examples of solid-lipid nanoparticles. They are cage-like 40 nm supramolecular assemblies comprising of phospholipid, cholesterol, and hydrophobic antigens and are used mostly as immunoadjuvants. For instance, ISCOMs are used to prolong blood-plasma levels of subcutaneously injected cyclosporine.

Microspheres and micro-capsules, as described herein, are yet other delivery devices that can be used to deliver the compositions disclosed herein. In contrast to liposomal delivery systems, microspheres and micro-capsules typically do not have an aqueous core but a solid polymer matrix or membrane. These delivery devices are obtained by controlled precipitation of polymers, chemical cross-linking of soluble polymers, and interfacial polymerization of two monomers or high-pressure homogenization techniques. The encapsulated compound is gradually released from the depot by erosion or diffusion from the particles. Successful formulations of short acting peptides, such as LHRH agonists like leuprorelin and triptoreline, have been developed. Poly(lactide co-glycolide (PLGA) microspheres are currently used as monthly and three monthly dosage forms in the treatment of advanced prostrate cancer, endometriosis, and other hormone responsive conditions. Leuprolide, an LHRH superagonist, was incorporated into a variety of PLGA matrices using a solvent extraction/evaporation method. As noted, all of these delivery devices can be used in the methods disclosed herein.

Pulmospheres are still other examples of delivery devices that can be used herein. Pulmospheres are hollow porous particles with a low density (less than about 0.1 gm/mL). Pulmospheres typically have excellent re-dispersibility and are usually prepared by supercritical fluid condensation technology. Co-spray-drying with certain matrices, such as carbohydrates, human serum albumin, etc., can improve the stability of proteins and peptides (*e.g*., insulin) and other biomolecules for pulmonary delivery. This type of delivery could be also accomplished with micro-emulsions and lipid emulsions, which are ultra fine, thin, transparent oil-in-water (o/w) emulsions formed spontaneously with no significant input of mechanical energy. In this technique, an emulsion can be prepared at a temperature, which must be higher than the phase inversion temperature of the system. At elevated temperature the emulsion is of water-in-oil (w/o) type and as it cools at the phase inversion temperature, this emulsion is inverted to become o/w. Due to their very small inner phase, they are extremely stable and used for sustained release of steroids and vaccines. Lipid emulsions comprise a neutral lipid core (*i.e*., triglycerides) stabilized by a monolayer of amphiphilic lipid (*i.e*., phospholipid) using surfactants like egg lecithin triglycerides and miglyol. They are suitable for passive and active targeting.

There are other oral delivery systems under investigation that are based on osmotic pressure modulation, pH modulation, swelling modulation, altered density and floating systems and mucoadhesiveness. These formulations and time-delayed formulations to deliver drugs in accordance with circadian rhythm of disease that are currently in use or investigation can be applied for delivery of the compositions disclosed herein.

In one particular aspect disclosed herein, the disclosed compounds, including nutritional supplement and pharmaceutical formulations thereof, can be incorporated into microcapsules as described herein.

In one aspect disclosed herein, the disclosed compounds can be incorporated into microcapsules. In one aspect, the microcapsule comprises an agglomeration of primary microcapsules and the chromium compounds described herein, each individual primary microcapsule having a primary shell, wherein the chromium compound is encapsulated by the primary shell, wherein the agglomeration is encapsulated by an outer shell. These microcapsules are referred to herein as "multicore microcapsules."

In another aspect, described herein are microcapsules comprising a chromium compound, a primary shell, and a secondary shell, wherein the primary shell encapsulates the chromium compound, and the secondary shell encapsulates the loading substance and primary shell. These microcapsules are referred to herein as "single-core microcapsules.

Optionally, other loading substances can be encapsulated with the chromium compound. The loading substance can be any substance that is not entirely soluble in the aqueous mixture. In one aspect, the loading substance is a solid, a hydrophobic liquid, or a mixture of a solid and a hydrophobic liquid. In another aspect, the loading substance comprises a grease, an oil, a lipid, a drug (*e.g*., small molecule), a biologically active substance, a nutritional supplement (*e.g*., vitamins), a flavour compound, or a mixture thereof. Examples of oils include animal oils (*e.g*., fish oil, marine mammal oil), vegetable oils (*e.g*., canola or rapeseed), mineral oils, derivatives thereof or mixtures thereof. The loading substance can be a purified or partially purified oily substance such as a fatty acid, a triglyceride or ester thereof, or a mixture thereof. In another aspect, the loading substance can be a carotenoid (*e.g*., lycopene), a satiety agent, a flavor compound, a drug (*e.g*., a water insoluble drug), a particulate, an agricultural chemical (*e.g*., herbicides, insecticides, fertilizers), or an aquaculture ingredient (*e.g*., feed, pigment).

In one aspect, the loading substance can be an omega-3 fatty acid. Examples of omega-3 fatty acids include α-linolenic acid (18:3ω3), octadecatetraenoic acid (18:4ω3), eicosapentaenoic acid (20:5ω3) (EPA), docosahexaenoic acid (22:6ω3) (DHA), docosapentaenoic acid (22:5ω3) (DPA), eicosatetraenoic acid (20:4ω3), uncosapentaenoic acid (21:5ω3), docosapentaenoic acid (22:5ω3) and derivatives thereof and mixtures thereof. Many types of derivatives of omega-3 fatty acids are well known in the art. Examples of suitable derivatives include, esters, such as phytosterol esters, branched or unbranched C₁-C₃₀ alkyl esters, branched or unbranched C₂-C₃₀ alkenyl esters, or branched or unbranched C₃-C₃₀ cycloalkyl esters such as phytosterol esters and C₁-C₆ alkyl esters. Sources of oils can be derived from aquatic organisms (*e.g*., anchovies, capelin, Atlantic cod, Atlantic herring, Atlantic mackerel, Atlantic menhaden, salmonids, sardines, shark, tuna, etc) and plants *(e.g.,* flax, vegetables, etc) and microorganisms (*e.g*., fungi and algae).

In one aspect, the loading substance can contain an antioxidant. Examples of antioxidants include vitamin E, CoQ₁₀, tocopherols, lipid soluble derivatives of more polar antioxidants such as ascorbyl fatty acid esters (*e.g*., ascorbyl palinitate), plant extracts (*e.g*., rosemary, sage and oregano oils), algal extracts, and synthetic antioxidants (*e.g*., BHT, TBHQ, ethoxyquin, alkyl gallates, hydroquinones, tocotrienols).

A number of different polymers can be used to produce the shell layers of the single and multicore microcapsules. Examples of such polymers include, but are not limited to, a protein, a polyphosphate, a polysaccharide, or a mixture thereof. In another aspect, the shell material used to prepare the single- and multicore microcapsules further comprises In another aspect, the shell material used to prepare the single- and multicore microcapsules further comprises gelatin type A, gelatin type B, polyphosphate, gum arabic, alginate, chitosan, carrageenan, pectin, starch, modified starch, alfa-lactalbumin, beta-lactoglobumin, ovalbumin, polysorbiton, maltodextrins, cyclodextrins, cellulose, methyl cellulose, ethyl cellulose, hydropropylmethylcellulose, carboxymethylcellulose, milk protein, whey protein, soy protein, canola protein, albumin, chitin, polylactides, polylactide-co-glycolides, derivatized chitin, chitosan, poly-lysine, various inorganic-organic composites, or any mixture thereof. It is also contemplated that derivatives of these polymers can be used as well. In another aspect, the polymer can be kosher gelatin, non-kosher gelatin, Halal gelatin, or non-Halal gelatin.

In one aspect, one or more of the shell layers in the single and multicore microcapsules comprises gelatin having a Bloom number less than 50. This gelatin is referred to herein as "low Bloom gelatin." The Bloom number describes the gel strength formed at 10 °C with a 6.67% solution gelled for 18 hours. In one aspect, the low Bloom gelatin has a Bloom number less than 40, less than 30, less than 20, or less than 10. In another aspect, the gelatin has a Bloom number of 45, 40, 35, 30, 25, 20, 15, 10, 9, 8, 7, 6, 5, 4, 3, 2, 1, or 0, where any two values can be used to produce a range. In another aspect, the low Bloom gelatin is in both the primary shell and the outer shell of the multicore microcapsule. In one aspect, the low Bloom gelatin is gelatin type A. In another aspect, the low Bloom gelatin is gelatin type A produced by Kenney & Ross Ltd., R.R. #3 Shelburne, NS Canada. In another aspect, gelatin having a Bloom number of zero is in both the primary shell and the outer shell of the multicore microcapsule.

In one aspect, the material used to make the shells of the single- or multicore microcapsules is a two-component system made from a mixture of two different types of polymers. In one aspect, the material is a complex coacervate between the polymer components. Complex coacervation is caused by the interaction between two oppositely charged polymers. In one aspect, the shell material used to produce the single and multicore microcapsules is composed of (1) low Bloom gelatin and (2) gelatin type B, polyphosphate, gum arabic, alginate, chitosan, carrageenan, pectin, carboxymethylcellulose, whey protein, soy protein, canola protein, albumin, or a mixture thereof. The molar ratio of the different polymers can vary. For example, the molar ratio of low Bloom gelatin to the other polymer component is from 1:5 to 15:1. For example, when low Bloom gelatin and polyphosphate are used, the molar ratio of low Bloom gelatin to polyphosphate is 8:1 to 12:1; when low Bloom gelatin and gelatin type B are used, the molar ratio is 2:1 to 1:2; and when low Bloom gelatin and alginate are used, the molar ratio is 3:1 to 8:1.

Processing aids can be included in the shell material (*e.g*., primary or outer shells). Processing aids can be used for a variety of reasons. For example, they may be used to promote agglomeration of the primary microcapsules, stabilize the emulsion system, improve the properties of the outer shells, control microcapsule size and/or to act as an antioxidant. In one aspect, the processing aid can be an emulsifier, a fatty acid, a lipid, a wax, a microbial cell (*e.g*., yeast cell lines), a clay, or an inorganic compound (*e.g*., calcium carbonate). Not wishing to be bound by theory, these processing aids can improve the barrier properties of the microcapsules. In one aspect, one or more antioxidants can be added to the shell material. Antioxidant properties are useful both during the process (*e.g*. during coacervation and/or spray drying) and in the microcapsules after they are formed (*i.e*. to extend shelf-life). Preferably a small number of processing aids that perform a large number of functions can be used. In one aspect, the antioxidant can be a phenolic compound, a plant extract, or a sulphur-containing amino acid. In one aspect, ascorbic acid (or a salt thereof such as sodium or potassium ascorbate) can be used to promote agglomeration of the primary microcapsules, to control microcapsule size and to act as an antioxidant. The antioxidant can be used in an amount of 100 ppm to 12,000 ppm, or from 1,000 ppm to 5,000 ppm. Other processing aids such as, for example, metal chelators, can be used as well. For example, ethylene diamine tetraacetic acid can be used to bind metal ions, which can reduce the catalytic oxidation of the loading substance.

In one aspect, the primary microcapsules (primary shells) have an average diameter of 40 nm to 10 µm, 0.1 µm to 10 µm, 1 µm to 10 µm, 1 µm to 8 µm, 1 µm to 6 µm, 1 µm to 4 µm, or 1 µm to 2 µm, or 1 µm. In another aspect, the multicore microcapsules can have an average diameter of from 1 µm to 2000 µm, 20 µm to 1000 µm, from 20 µm to 100 µm, or from 30 µm to 80 µm. In another aspect, the single-core microcapsules have an outer diameter of from 1 µm to 2,000 µm.

The microcapsules described herein generally have a combination of high payload and structural strength. For example, payloads of loading substance can be from 20% to 90%, 50% to 70% by weight, or 60% by weight of the single or multicore microcapsules.

In one aspect, the methods disclosed in U.S. Patent Application Publication No. 2003/0193102 can be used to encapsulate the chromium compounds described herein. It is also contemplated that one or more additional shell layers can be placed on the outer shell of the single or multicore microcapsules. In one aspect, the techniques described in International Publication No. WO 2004/041251 A1 can be used to add additional shell layers to the single and multicore microcapsules.

### Targeted delivery

The disclosed liposomes and microcapsules can be targeted to a particular cell type, such as islets cells, via antibodies, receptors, or receptor ligands. The following references are examples of the use of this technology to target specific tissue (Senter, et al., Bioconjugate Chem 2:447-51, 1991; Bagshawe, Br J Cancer 60:275-81, 1989; Bagshawe, et al., Br J Cancer 58:700-3, 1988; Senter, et al., Bioconjugate Chem 4:3-9, 1993; Battelli, et al., Cancer Immunol Immunother 35:421-5, 1992; Pietersz and McKenzie, Immunolog Reviews 129:57-80, 1992; and Roffler, et al., Biochem Pharmacol 42:2062-5, 1991). These techniques can be used for a variety of other specific cell types.

### Foodstuffs

Also disclosed herein are foodstuffs comprising any of the microcapsules and emulsions disclosed herein. By "foodstuff' is meant any article that can be consumed (*e.g*., eaten, drank, or ingested) by a subject. In one aspect, the microcapsules can be used as nutritional supplements to a foodstuff. For example, the microcapsules and emulsions can be loaded with vitamins, omega-3 fatty acids, and other compounds that provide health benefits. In one aspect, the foodstuff is a baked good, a pasta, a meat product, a frozen dairy product, a milk product, a cheese product; an egg product, a condiment, a soup mix, a snack food, a nut product, a plant proteins product, a hard candy, a soft candy, a poultry product, a processed fruit juice, a granulated sugar (*e.g.,* white or brown), a sauce, a gravy, a syrup, a nutritional bar, a beverage, a dry beverage powder, a jam or jelly, a fish product, or pet companion food. In another aspect, the foodstuff is bread, tortillas, cereal, sausage, chicken, ice cream, yogurt, milk, salad dressing, rice bran, fruit juice, a dry beverage powder, rolls, cookies, crackers, fruit pies, or cakes.

### Methods of use:

The compounds disclosed herein also have a wide variety of uses, In the disclosed compounds, the one or more fatty acids are bonded to a benzenediol derivative and are therefore an integral part of the complex. Thus, while not wishing to be bound by theory, it is believed that the fatty acids play at least two roles, *i.e.,* they make the benzenediol biologically available and they also contribute with their inherent biological activity. Thus, the disclosed compounds (including the nutritional supplements, pharmaceutical formulations, microcapsules, liposomes, and foodstuffs) can deliver fatty acids (*e.g.,* omega-3 fatty acids), lowering triglycerides and influencing prevention or treatment of neurodegenerative diseases (Calon, et al., Neuron 43:633-45, 2004), and benzenediol derivatives like CoQ₁₀, a cofactor with a beneficial effect on cardiovascular and central nervous system health.

In one particular aspect, disclosed herein are methods of lowering total cholesterol levels, triglyceride levels, and increasing HDL levels, or a combination thereof in a subject by administering an effective amount of any of the compounds described herein *(e.g.,* Formula IV) to the subject In still another aspect, disclosed herein are methods of improving insulin sensitivity in a subject by administering an effective amount of any of the compounds described herein to the subject. In a further aspect, disclosed herein are methods of reducing hyperglycemia in a subject by administering an effective amount of any of the compounds described herein to the subject In yet another aspect, disclosed herein axe methods of reducing hypercholesterolemia in a subject by administering an effective amount of any of the compounds described herem to the subject.

Also disclosed herein, in one aspect, are methods of preventing a mitochondrial condition or disease in a subject by administering an effective amount of any of the compounds described herein to the subject. An example of a mitochondrial condition includes, but is not limited to, mitochondriopathy. Mitochondriopathy can be characterized by a CoQ₁₀ deficiency, ubiquinone-cytochrome c oxidoreductase deficiency, cytochrome c oxidase deficiency, chronic progressive external ophthalmoplegia syndrome, age-related macular degeneration, neuropathy, ataxia, or retinitis Pigmentosa.

In another aspect, disclosed herein are methods of increasing circulation in a subject by administering an effective amount of any compound comprising any of the compounds described herein to the subject. In still another aspect, disclosed herein are methods of increasing the immune system in a subject by administering an effective amount of any compound comprising any of the compounds described herein to the subject. Principles and examples of use of immunostimulants and immunomodulators are described for instance in: "Immunostimulants now and tomorrow" (Azuma I, Jolles G, eds.), Japan ScientificSocieties Press, Tokyo, 1987; Hadden JW (1992) "Classification of immunotherapeutic agents. In: Developments of Biological Standardization," Vol. 77, (eds Brown F, Revillard JP): 5 -15; Karger, Basel; Galeotti M (1998) "Some aspects of the application of immunostimulants and a critical review of methods for their evaluation" JAppl Ichtuol 189-199*;* Halperin SA, Smith BS, Nolan C, Shay J, Kralovec J (2003) "Randomized, double-blind, placebo-controlled trial of the safety and immunostimmulatory effect of a Chlorilla-derived food supplement in healthy adults undergoing influenza immunization" Can Med Assoc J 169: 111-117), In yet another aspect, disclosed herein are methods of reducing the side effects of chemotherapy in a subject by administering an effective amount of any compound comprising any of the compounds described herein to the subject. In still another aspect, disclosed herein are methods of treating or preventing degenerative heart disease in a subject by administering an effective amount of any compound comprising any of the compounds described herein to the subject.

Further, disclosed herein are methods of treating other conditions or diseases in a subject by administering an effective amount of any compound comprising Formula I to the subject. Such other conditions or diseases include, but are not limited to, cystic fibrosis, asthma, periodontal (gum) disease. Alzheimer's disease, poor athletic performance, breast cancer, chronic obstructive pulmonary disease (COPD), HIV, male infertility, insulin resistance syndrome (Syndrome X), lung cancer, and prostate cancer.

The disclosed compounds herein can be used neat or in combination with some other component. For example, the compounds can be used in the disclosed methods in the form of any of the nutritional supplements disclosed herein. In another example, the compounds can be used in the disclosed methods in the form of any of the pharmaceutical formulations disclosed herein. In still another example, the compounds can be encapsulated in any of the microcapsules or liposomes disclosed herein, or incorporated into any foodstuff disclosed herein and used in the disclosed methods.

it is contemplated that the methods disclosed herein can be accomplished by administering various forms of the compound disclosed, herein. For example, one can administer any of the pharmaceutical formulations with any of the foodstuffs disclosed herein. In another example; one can administer any of the microcapsules with any of the nutritional supplements disclosed herein. In yet another example, one can administer any of the pharmaceutical formulations with any of the microcapsules and nutritional supplement disclosed herein,

### Dosage

When used in the above described methods or other treatment, or in the nutritional supplements, pharmaceutical formulations, microcapsules; liposomes, or foodstuffs disclosed herein, an "effective amount" of one of the disclosed compounds can be employed in pure form or, where such forms exist, in pharmaceutically acceptable salt form and with or without a pharmaceutically acceptable excipient, carrier, or other additive.

The specific effective dose level for any particular subject will depend upon a variety of factors including the condition or disease being treated and the severity of the condition or disease; activity of the specific compound employed; the specific composition employed; the age, body weight, general health, sex and diet of the subject; the time of administration; the route of administration; the rate of excretion of the specific compound employed; the duration of the treatment; drugs used in combination or coincidential with the specific compound employed and like factors well known in the medical arts. For example, it is well within the skill of the art to start doses of the compound at levels lower than those required to achieve the desired therapeutic effect and to gradually increase the dosage until the desired effect is achieved. If desired, the effective daily dose may be divided into multiple doses for purposes of administration. Consequently, single dose compositions may contain such amounts or submultiples thereof to make up the daily dose.

The dosage can be adjusted by the individual physician or the subject in the event of any counterindications. Dosage can vary, and can be administered in one or more dose administrations daily, for one or several days. Guidance can be found in the literature for appropriate dosages for given classes of pharmaceutical products. A typical daily dosage of the compounds disclosed herein used alone might range from 10mg to up to 500 mg (benzenediol content only) or more per day, depending on the factors mentioned above.

### Administration and delivery

In one aspect, disclosed herein are uses of a microcapsule to deliver a loading substance to a subject, wherein the microcapsule contains any of the compounds disclosed herein. Also disclosed are methods for delivering a compound composting Formula IV to a subject by administering to the subject any of the microcapsules disclosed herein. Further, disclosed are methods for delivering a compound disclosed herein to a subject by administering to the subject any of the nutritional supplements, pharmaceutical formulations, liposomes, and/or foodstuffs disclosed herein.

The compounds disclosed herein (including nutritional supplements, microcapsules, liposomes, and pharmaceutical formulation) can be administered orally, parenterally *(e.g.,* intravenously), by intramuscular injection, by intraperitoneal injection, tramsdermally, extracorporeally or topically including topical intranasal administration or administration by inhalant. As used herein, "topical intranasal administration" means delivery of the compositions into the nose and nasal passages through one or both of the nares and can comprise delivery by a spraying mechanism or droplet mechanism, or through aerosolization of the nucleic acid or vector Administration of the compositions by inhalant can be through the nose or mouth via delivery by a spraying or droplet mechanism. Delivery can also be directly to any area of the respiratory system (*e.g.,* lungs) via incubation.

### EXAMPLES

The following examples are set forth below to illustrate the methods and results according to the disclosed subject matter. These examples are not intended to be inclusive of all aspects of the subject matter disclosed herein, but rather to illustrate representative methods and results.

Efforts have been made to ensure accuracy with respect to numbers (*e.g.,* amounts, temperature, etc.) but some errors and deviations should be accounted for. Unless indicated otherwise, parts are parts by weight, temperature is in °C or is at ambient temperature, and pressure is at or near atmospheric. There are numerous variations and combinations of reaction conditions, e.g., component concentrations, desired solvents, solvent mixtures, temperatures, pressures and other reaction ranges and conditions that can be used to optimize the product purity and yield obtained from the described process. Only reasonable and routine experimentation will be required to optimize such process conditions.

### Example 1: Physical Blending of CoQ₁₀ and Fish Oil or Fish Oil Concentrates

CoQ₁₀ was solubilized in 0355 EE (fish oil concentrate in ethylester form containing 3% EPA and 55% DHA), 4020EE (fish oil concentrate in ethylester form containing 40% EPA and 20% DHA) and 1812TG (purified fish oil containing 18% EPA and 12% of DHA) and the final solubility was examined under different conditions and time of storage. All of these starting oils were manufactured by Ocean Nutrition Canada, Mulgrave, NS). The basic results are listed in the Table 4.

| Table 4. Solubility of CoQ₁₀ₒₓ in selected fish oil products | | | |
|---|---|---|---|
| | Solubility of CoQ₁₀ in 0355EE | Solubility of CoO₁₀ in 4020EE. | Solubility of CoQ₁₀ in 1812TG |
| | No Heating Prior Storage | Heating Prior Storage | Heating Prior Storage |
| Solubility/Storage | Confirmed solubility. (%,w/w) | Confirmed solubility. (%,w/w) | Confirmed solubility. (%,w/w) |
| Solubility at RT | 6.6 | ND | ND |
| Storage at 4°C for24h | 2.5 | ND | ND |
| Solubility at 50°C | 27% | 9 | 11 |
| Storage at 4°C | 3.2% | 5.7 | <2.9 |

Solubilization of CoQ₁₀ₒₓ in the tested fish oil and concentrates is clearly very limited.

### Example 2: Direct Esterification of CoQ₁₀

CoQ₁₀ exists primarily in its oxidized ubiquinone form (CoQ₁₀ₓ). However, it was tested whether the concentration of the reduced form in a normal CoQ₁₀ₒₓ. sample would be high enough to initiate the coupling and progressively form more and more conjugate due to the equilibrium shift, (*e.g.,* that the reaction would be under thermodynamic control). Although it is generally accepted that for acylation of phenols, basic catalysis is more effective, these reactions were performed using both acidic and basic catalysis, initially with NaHSO₄ or K₂CO₃, respectively. The molar ratio of free fatty acid over CoQ₁₀ initially used was 2:1 and 5:1, respectively, and the reactions were performed at 120°C. An H₃BO₃/H₂SO₄ combination and carbodiimides were also used, including PS-carbodiimide, a resin bound coupling agent, (both at room temperature and under reflux).

To improve the yields of the acylation, CoQ₁₀ₒₓ was converted to the corresponding reduced form (CoQ_{10red}) by NaBH₄, and later even more conveniently by hydrogen generated by Zn from AcOH. High yields were achieved by reacting CoQ₁₀ with free fatty acid that was first converted to the corresponding chloride with SOCl₂. It was found that the use of nitrogen base such as Et₃N was helpful to achieve good conversions. The highest yields were accomplished using P₂O₅ (phosphoric acid anhydride). While not wishing to be bound by theory, it was assumed that a mixed anhydride of the formula (DHA-COO)₂PO is the reaction intermediate. In addition, excess catalyst can drive the equilibrium by capturing the produced water.

### Example 3: Preparation of CoQ_{10red}

Ten grams (11.57 mmol) ofCo_{Q10ox} was stirred with 20 g zinc powder in 200 mL glacial acetic acid under reflux for 1 hour (h) at 65-70°C. The residual acetic acid was then evaporated and the mixture extracted 3 times with hexane, centrifuged, filtered and concentrated. The product was obtained in a quantitative yield, in a form of light yellow syrup that quickly crystallized yielding white crystals. The product was stored under nitrogen.

### Preparation of Omega-3-CoQ₁₀ Conjugates

### Example 4

A mixture of 3.93 g (11.57 mmol) 4020FFA (Free Fatty Acid, prepared by hydrolysis of 4020EE, a product of Ocean Nutrition Canada, Mulgrave, NS) with 10 mL thionyl chloride was refluxed until no more HCl was produced (approximately 2 h) and then excess thionyl chloride was evaporated. The product (FFA-Cl) was in a form of dark brown liquid and obtained in a quantitative yield.

A mixture of CoQ_{10red} (10 g, 11.57 mmol) and triethylamine (1.627 mL,1.171 g, 11.57 mmol) in 10 mL CH₂Cl₂ was added to the prepared FFA-Cl. The reaction was stirred for 5 h at room temperature and pressure with a CaCl₂ trap, then the solvent was evaporated, and the residue was mixed with 60 mL acetone to precipitate the triethylammonium chloride. The precipitate was filtered off, acetone evaporated, and the product reconstituted in hexane. The reaction was monitored by TLC (hexane/diethyl ether (6:4) +1% AcOH, and sprayed with 15% H₂SO₄ in MeOH). The product was isolated from the final reaction mixture by column chromatography on silica gel using 1 % diethylether in hexane as a solvent. It was obtained in a form of a yellow liquid and stored in the refrigerator under nitrogen.

### Example 5

A mixture of 5.62 g (18 mmol) of 4020FFA and 3.71 g dicyclohexyldicarbodimide (DCC) (18 mmol) in 18 mL of hexane was stirred overnight. Then a mixture of CoQ_{10red} (0.865 g, 1 mmol) and triethylamine (0.276 ml, 0.2024 g, 2 mmol) was added to a portion of the activated free fatty acid (FFA) (0.624 g, 2 mmol). The reaction was stirred overnight at room temperature, monitored and the product isolated as described in Example 4.

### Example 6

A solution of reduced CoQ_{10red} (2.0 g, 2.3 mmol) in 4020FFA (1.7 g 5 mmol) was prepared at 50°C and P₂O₅ (1.42 g, 10 mmol) was added. The reaction was then heated at 50-60°C for 28 h under vacuum (< 7 mbar). The reaction mixture was removed from the vessel using a mixture of hexane and chloroform; the extract was evaporated and then chromatographed as described in Example 4.

### Example 7: Analysis

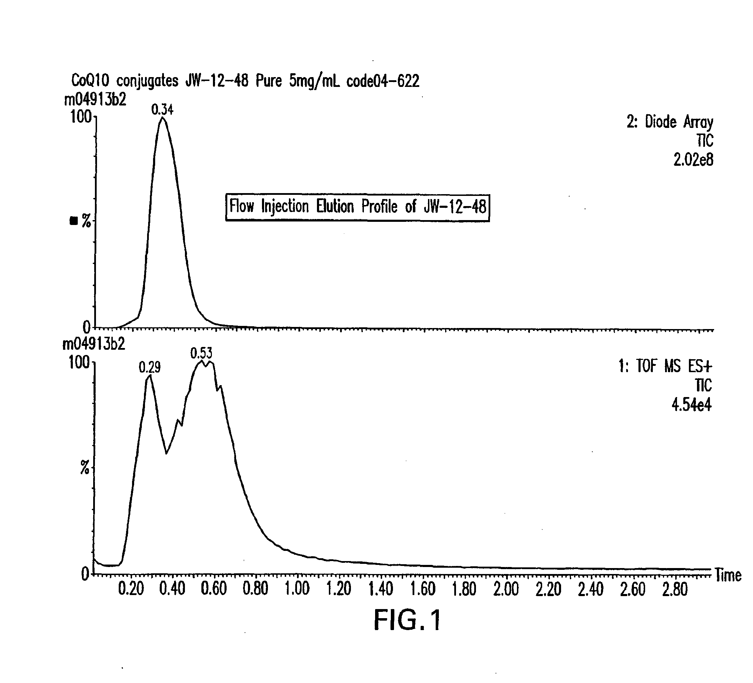

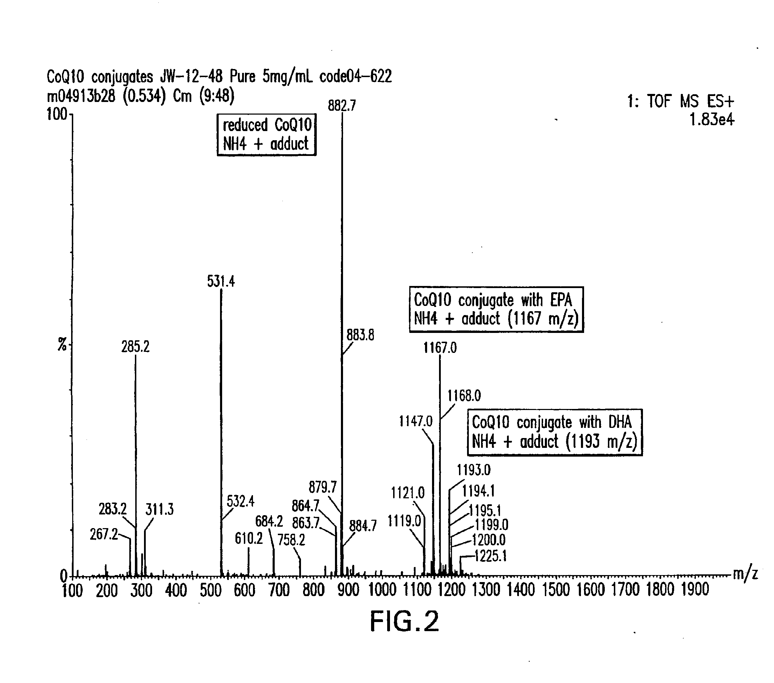

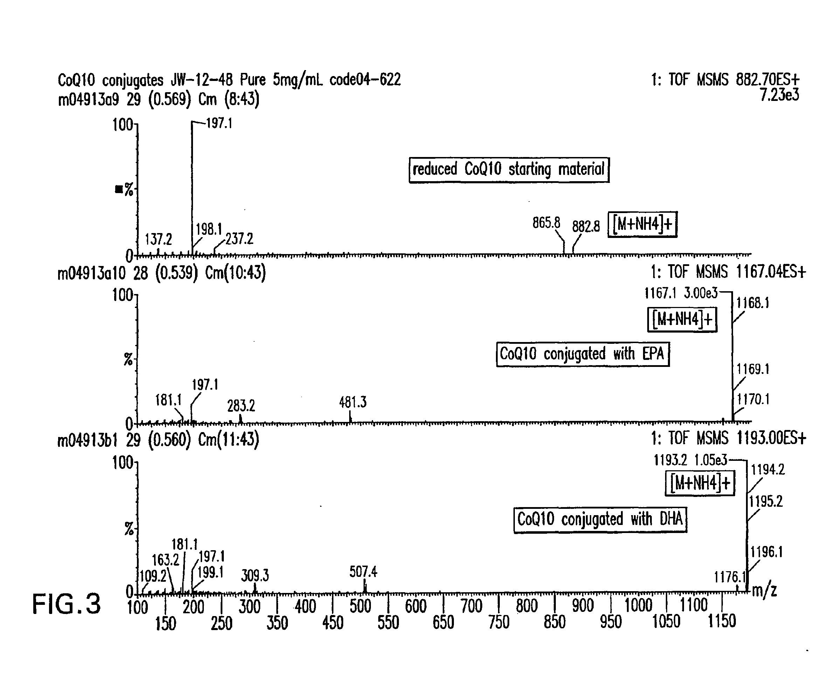

Mass spectral analysis confirmed synthesis of bifunctional omega-3-CoQ₁₀ conjugate. A sample of the reaction mixture was injected directly into Micromass QTOF mass spectrometer chromatography using a Waters 2695 HPLC system. A Waters 996 photo diode array was installed upstream from the mass spectrometer to monitor the elution profile using ultraviolet absorbance. The eluent (40% heptane, 20% chloroform, 40% methanol with 0.1% AcONH₄) flow rate was set to 0.2 ml/min and the sample injection volume was 5 µL. The mass spec was operated in ESI+ mode to monitor for cationic ammonium adducts of the sample. There was significant evidence for CoQ_{10red} conjugated with both EPA and DHA fatty acids as evidenced from Figs. 1-3.

## Claims

1. A compound having Formula IV 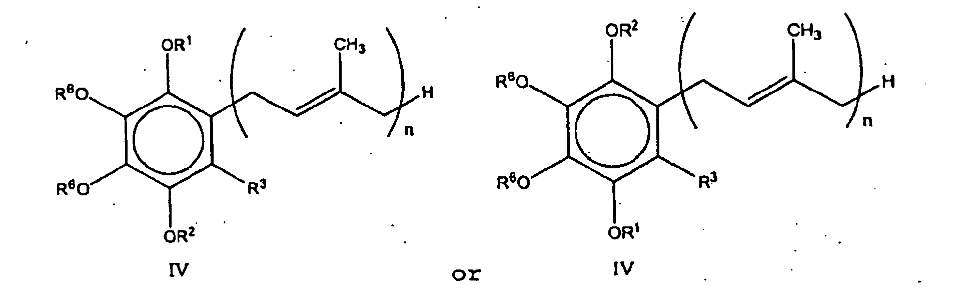 wherein R¹ is an omega-3 unsaturated fatty acid residue; R² is hydrogen or an omega-3 unsaturated fatty acid residue; R³ and each R⁶ is methyl and n is 10.

2. The compound of claim 1, wherein R¹ is an omega-3 unsaturated fatty acid residue derived from fish oil.

3. The compound of claim 1, wherein R¹ is an unsaturated fatty acid residue derived from eicosapentaenoic acid 20:5ω3 (EPA) docosahexaenoic acid 22:6ω3 (DHA), docosapentaenoic acid 22:5ω3 (DPA); or a combination thereof.

4. The compound of claim 1, wherein R¹ and R² are unsaturated fatty acid residues derived from eicosapentaenoic acid 20 : 5ω3 (EPA) , docosahexaenoic acid 22:6ω3 (DHA), docosapentaenoic acid 22:5ω3 (DPA), or a combination thereof.

5. A nutritional supplement comprising a compound in any of claims 1 to 4.

6. The nutritional supplement of claim 5, comprising from 0.05% to 20% by weight of the compound.

7. The nutritional supplement of claim 5, comprising from 1% to 7.5% by weight of the compound.

8. The nutritional supplement of claim 5, wherein the supplement comprises less than or equal to 100% by weight of the compound.

9. The nutritional supplement of claim 5, wherein the supplement is in the form of a tablet, gel-cap, capsule, liquid, or syrup.

10. A delivery device comprising a compound in any of claims 1 to 4.

11. The delivery device of claim 10, wherein the device comprises a microcapsule, a liposome, noisome, nanoerythrosome, solid-liquid nanoparticle, microsphere, or pulmosphere.

12. The delivery device of claim 10, wherein the device comprises a microcapsule, wherein the microcapsule comprises an agglomeration of primary microcapsules, each individual primary microcapsule having a primary shell and the agglomeration being encapsulated by an outer shell, wherein the compound in any of claims 1 to 4 is encapsulated in the primary microcapsule.

13. The delivery device of claim 12, wherein the primary shell and the outer shell comprises comprises gelatin type A, gelatin type B, polyphosphate, gum arabic, alginate, chitosan, carrageenan, pectin, starch, modified starch, alfa-lactalbumin, beta-lactoglobumin, ovalbumin, polysorbitol, maltodextrins, cyclodextrins, cellulose, methyl cellulose, ethyl cellulose, hydroxypropylmethylcellulose, carboxymethylcellulose, milk protein, whey protein, soy protein, canola protein, albumin, chitin, polylactide, poly-lactide-co-glycolide, polylysine, kosher gelatin, non-kosher gelatin, Halal gelatin, non-Halal gelatin, or a mixture thereof.

14. The delivery device of claim 12, wherein the primary shell and the outer shell comprises gelatine type A having a Bloom strength of from 0 to 350.

15. The delivery device of claim 12, wherein the primary shell and the outer shell comprises a no bloom fish gelatin.

16. The delivery device of claim 12', further comprising an additional shell surrounding the outer shell, wherein at least one of the primary, outer, and additional shells comprise a complex coacervate.

17. A foodstuff comprising the compound in any of claims 1 to 4.

18. The foodstuff of claim 17, wherein the foodstuff is a baked good, a pasta, a meat product, a frozen dairy product, a milk product, a cheese product, an egg product, a condiment, a soup mix, a snack food, a nut product, a plant protein product, a hard candy, a soft candy, a poultry product, a processed fruit juice, a granulated sugar, a sauce, a gravy, a syrup, a nutritional bar, a beverage, a dry beverage powder; a jam or jelly, a fish product, or pet companion food.

19. The foodstuff of claim 17, wherein the foodstuff is bread, tortillas, cereal, sausage, chicken, ice cream, yogurt, milk, salad dressing, rice bran, fruit juice, a dry beverage powder, rolls, cookies, crackers, fruit pies, or cakes.

20. Use of an effective amount of the compound in any of claims 1 to 4 in the manufacture of a medicament for lowering total cholesterol levels or triglyceride levels, increasing HDL levels, or a combination thereof in a subject.

21. Use of an effective amount of the compound in any of claims 1 to 4 in the manufacture of a medicament for one or more of the following:
i. reducing hyperglycemia in a subject;
ii. reducing hypercholesterolemia in a subject;
iii. increasing circulation in a subject;
iv. increasing the immune system in a subject;
v. reducing the side effects of chemotherapy in a subject;
vi. treating or preventing degenerative heart disease in a subject.

22. Use of an effective amount of the compound in any of claims 1 to 4 in the manufacture of a medicament for treating or preventing a mitochondrial condition or disease in a subject.

23. The use of claim 22, wherein the condition is mitochondriopathy.

24. The use of claim 23, wherein the mitochondriopathy is Coenzyme Q₁₀ deficiency, ubiquinone-cytochrome c oxidoreductase deficiency, cytochrome c oxidase deficiency, chronic progressive external ophthalmoplegia syndrome, age- related macular degeneration, neuropathy, ataxia, or retinitis Pigmentosa.

25. A pharmaceutical formulation comprising the compound in any of claims 1, to 4 and a pharmaceutical carrier.

## Patentansprüche

1. Verbindung mit der Formel IV 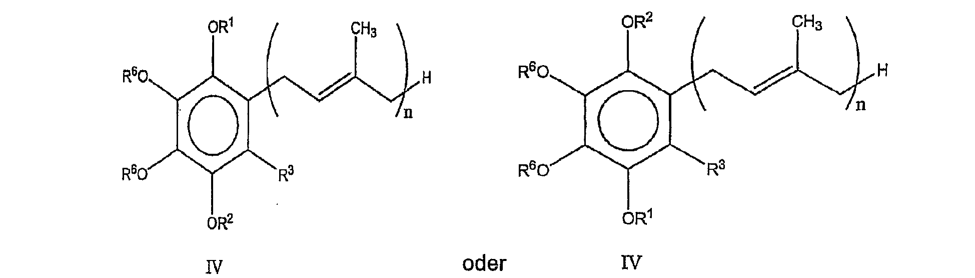 wobei R¹ ein Omega-3 ungesättigter Fettsäurerest ist; R² Wasserstoff oder ein Omega-3 ungesättigter Fettsäurerest ist; R³ und jeder R⁶ Methyl ist und n 10 ist.

2. Verbindung nach Anspruch 1, wobei R¹ ein Omega-3 ungesättigter Fettsäurerest ist, der von Fischöl abgeleitet ist.

3. Verbindung nach Anspruch 1, wobei R¹ ein ungesättigter Fettsäurerest ist, der von Eicosapentaensäure 20:5cm3 (EPA), Docosahexaensäure 22:6w3 (DHA), Docosapentaensäure 22:5w3 (DPA) oder einer Kombination davon abgeleitet ist.

4. Verbindung nach Anspruch 1, wobei R¹ und R² ungesättigte Fettsäurereste sind, die von Eicosapentaensäure 20:5cm3 (EPA), Docosahexaensäure 22:6ω3 (DHA), Docosapentaensäure 22:5cm3 (DPA) oder einer Kombination davon abgeleitet sind.

5. Nahrungsergänzungsmittel, umfassend eine Verbindung nach einem der Ansprüche 1 bis 4.

6. Nahrungsergänzungsmittel nach Anspruch 5, umfassend von 0,05 Gew.-% bis 20 Gew.-% der Verbindung.

7. Nahrungsergänzungsmittel nach Anspruch 5, umfassend von 1 Gew.-% bis 7,5 Gew.-% der Verbindung.

8. Nahrungsergänzungsmittel nach Anspruch 5, wobei das Ergänzungsmittel weniger als oder gleich 100 Gew.-% der Verbindung umfasst.

9. Nahrungsergänzungsmittel nach Anspruch 5, wobei das Ergänzungsmittel in Form einer Tablette, Gel-Kapsel, Kapsel, Flüssigkeit oder Sirup ist.

10. Freisetzungsvorrichtung, umfassend eine Verbindung nach einem der Ansprüche 1 bis 4.

11. Freisetzungsvorrichtung nach Anspruch 10, wobei die Vorrichtung eine Mikrokapsel, ein Liposom, Noisom, Nanoerythrosom, fest-flüssigen Nanopartikel, Mikrosphäre oder Pulmosphäre umfasst.

12. Freisetzungsvorrichtung nach Anspruch 10, wobei die Vorrichtung eine Mikrokapsel umfasst, wobei die Mikrokapsel eine Agglomeration von primären Mikrokapseln umfasst, wobei jede einzelne primäre Mikrokapsel eine primäre Hülle hat und die Agglomeration durch eine äußere Hülle eingekapselt ist, wobei die Verbindung nach einem der Ansprüche 1 bis 4 in die primäre Mikrokapsel eingekapselt ist.

13. Freisetzungsvorrichtung nach Anspruch 12, wobei die primäre Hülle und die äußere Hülle Typ A-Gelatine, Typ B-Gelatine, Polyphosphat, Gummi Arabicum, Alginat, Chitosan, Carrageenan, Pektin, Stärke, modifizierte Stärke, alpha-Lactalbumin, beta-Lactoglobumin, Ovalbumin, Polysorbitol, Maltodextrine, Cyclodextrine, Zellulose, Methylzellulose, Ethylzellulose, Hydroxypropylmethylzellulose, Carboxymethylzellulose, Milchprotein, Molkenprotein, Sojaprotein, Rapsprotein, Albumin, Chitin, Polylactid, Polylactid-co-Glycolid, Polylysin, koschere Gelatine, nicht-koschere Gelatine, Halal Gelatine, nicht-Halal Gelatine oder eine Mischung davon umfasst.

14. Freisetzungsvorrichtung nach Anspruch 12, wobei die primäre Hülle und die äußere Hülle Typ A-Gelatine mit einer Bloom Zahl von 0 bis 350 umfasst.

15. Freisetzungsvorrichtung nach Anspruch 12, wobei die primäre Hülle und die äußere Hülle eine nicht Bloom Fischgelatine umfasst.

16. Freisetzungsvorrichtung nach Anspruch 12, weiter umfassend eine zusätzliche Hülle, die die äußere Hülle umgibt, wobei mindestens eine der primären Hülle, der äußeren Hülle und der zusätzlichen Hülle ein komplexes Koazervat umfasst.

17. Nahrungsmittel, umfassend die Verbindung nach einem der Ansprüche 1 bis 4.

18. Nahrungsmittel nach Anspruch 17, wobei das Nahrungsmittel eine Backware, Nudeln, ein Fleischprodukt, ein gefrorenes Molkereiprodukt, ein Milchprodukt, ein Käseprodukt, ein Eiprodukt, eine Würze, eine Fertigsuppe, ein lmbiss, ein Nußprodukt, ein Pflanzenprotein-Produkt, ein hartes Bonbon, ein weiches Bonbon, ein Geflügelprodukt, ein verarbeiteter Fruchtsaft, ein Kristallzucker, eine Soße, ein Bratensaft, ein Sirup, ein Nährstoffriegel, ein Getränk, ein Trockengetränkepulver, eine Marmelade oder ein Gelee, ein Fischprodukt oder eine Tiernahrung ist.

19. Nahrungsmittel nach Anspruch 17, wobei das Nahrungsmittel Brot, Tortillas, Getreide, Wurst, Huhn, Eiscreme, Joghurt, Milch, Salatdressing, Reiskleie, Fruchtsaft, ein Trockengetränkepulver, Brötchen, Kekse, Cracker, Fruchtkuchen oder Kuchen ist/sind:

20. Verwendung einer wirksamen Menge der Verbindung nach einem der Ansprüche 1 bis 4 zur Herstellung eines Medikaments zur Senkung der Gesamtcholesterinmengen oder Triglyceridmengen, Erhöhung der HDL-Mengen oder einer Kombination davon in einem Individuum.

21. Verwendung einer wirksamen Menge der Verbindung nach einem der Ansprüche 1 bis 4 zur Herstellung eines Medikaments für eines oder mehrere der Folgenden:
i. Reduzieren von Hyperglykämie in einem Individuum;
ii. Reduzieren von Hypercholesterinämie in einem Individuum;
iii. Steigern des Kreislaufs in einem Individuum;
iv. Steigern des Imunsystems in einem Individuum;
v. Reduzieren der Nebenwirkungen von Chemotherapie in einem Individuum;
vi. Behandeln oder Vermeiden von degenerativer Herzerkrankung in einem Individuum.

22. Verwendung einer wirksamen Menge der Verbindung nach einem der Ansprüche 1 bis 4 zur Herstellung eines Medikaments zur Behandlung oder Vermeidung einer mitochondriellen Störung oder Erkrankung in einem Individuum.

23. Verwendung nach Anspruch 22, wobei die Störung Mitochondriopathie ist.

24. Verwendung nach Anspruch 23, wobei die Mitochondriopathie Coenzym Q₁₀-Mangel, Ubichinon-Cytochrom c Oxidoreductase-Mangel, Cytochrom c Oxidase-Mangel, chronisch progressives externes Ophthalmoplegie-Snydrom, altersbedingte Makuladegeneration, Neuropathie, Ataxie oder Retinitis pigmentosa ist.

25. Pharmazeutische Formulierung, umfassend die Verbindung nach einem der Ansprüche 1 bis 4 und einen pharmazeutischen Träger.

## Revendications

1. Composé de formule IV 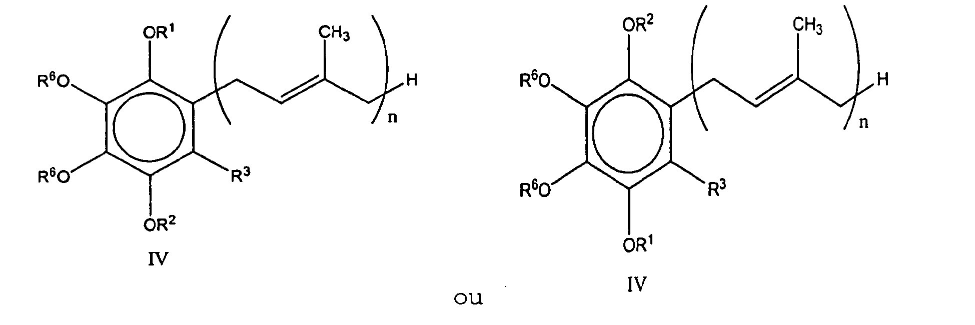 dans laquelle R¹ représente un résidu d'acide gras oméga-3 insaturé ; R² représente un atome d'hydrogène ou un résidu d'acide gras oméga-3 insaturé ; R³ et chaque groupe R⁶ représentent un groupe méthyle et n est égal à 10.

2. Composé suivant la revendication 1, dans lequel R¹ représente un résidu d'acide gras oméga-3 insaturé dérivé d'une huile de poisson.

3. Composé suivant la revendication 1, dans lequel R¹ représente un résidu d'acide gras insaturé dérivé d'acide eicosapentaénoïque 20:5ω3 (EPA), d'acide docosahexaénoïque 22:6ω3 (DHA), d'acide docosapentaénoïque 22:5ω3 (DPA) ou d'une de leurs associations.

4. Composé suivant la revendication 1, dans lequel R¹ et R² représentent des résidus d'acides gras insaturés dérivés de l'acide eicosapentaénoïque 20:5ω3 (EPA), de l'acide docosahexaénoïque 22:6ω3 (DHA), de l'acide docosapentaénoïque 22:5ω3 (DPA) ou d'une de leurs associations.

5. Supplément nutritionnel comprenant un composé de l'une quelconque des revendications 1 à 4.

6. Supplément nutritionnel suivant la revendication 5, comprenant 0,05 % à 20 % en poids du composé.

7. Supplément nutritionnel suivant la revendication 5, comprenant 1 % à 7,5 % en poids du composé.

8. Supplément nutritionnel suivant la revendication 5, ledit supplément comprenant une proportion inférieure ou égale à 100 % en poids du composé.

9. Supplément nutritionnel suivant la revendication 5, ledit supplément étant sous forme d'un comprimé, d'un gel-cap, d'une capsule, d'un liquide ou d'un sirop.

10. Dispositif d'administration comprenant un composé de l'une quelconque des revendications 1 à 4.

11. Dispositif d'administration suivant la revendication 10, ledit dispositif comprenant une microcapsule, un liposome, un noisome, un nanoérythrosome, une nanoparticule solide-liquide, une microsphère ou une pulmosphère.

12. Dispositif d'administration suivant la revendication 10, ledit dispositif comprenant une microcapsule, ladite microcapsule comprenant un agglomérat de microcapsules primaires, chaque microcapsule primaire individuelle ayant une enveloppe primaire et l'agglomérat étant encapsulé par une enveloppe extérieure, le composé de l'une quelconque des revendications 1 à 4 étant encapsulé dans la microcapsule primaire.

13. Dispositif d'administration suivant la revendication 12, dans lequel l'enveloppe primaire et l'enveloppe extérieure comprennent de la gélatine de type A, de la gélatine de type B, un polyphosphate, de la gomme arabique, un alginate, du chitosane, de la carraghénine, de la pectine, de l'amidon, un amidon modifié, de l'alpha-lactalbumine, de la bêta-lactoglobuline, de l'ovalbumine, du polysorbitol, des maltodextrines, des cyclodextrines, de la cellulose, de la méthylcellulose, de l'éthylcellulose, de l'hydroxypropylméthylcellulose, de la carboxyméthyl-cellulose, de la protéine du lait, de la protéine du petit lait, de la protéine de soja, de la protéine de canola, de l'albumine, de la chitine, un polylactide, un poly-lactide-co-glycolide, de la polylysine, de la gélatine cachère, de la gélatine non cachère, de la gélatine Halal, de la gélatine non Halal ou un de leurs mélanges.

14. Dispositif d'administration suivant la revendication 12, dans lequel l'enveloppe primaire et l'enveloppe extérieure comprennent de la gélatine de type A ayant un degré Bloom de 0 à 350.

15. Dispositif d'administration suivant la revendication 12, dans lequel l'enveloppe primaire et l'enveloppe extérieure comprennent une gélatine de poisson ayant un degré Bloom nul.

16. Dispositif d'administration suivant la revendication 12, comprenant en outre une enveloppe supplémentaire entourant l'enveloppe extérieure, dans lequel au moins une des enveloppes consistant en l'enveloppe primaire, l'enveloppe extérieure et l'enveloppe supplémentaire comprend un produit de coacervation complexe.

17. Denrée alimentaire comprenant le composé de l'une quelconque des revendications 1 à 4.

18. Denrée alimentaire suivant la revendication 17, ladite denrée alimentaire étant une denrée cuite au four, des pâtes alimentaires, un produit à base de viande, un produit laitier congelé, un produit laitier, un produit du type du fromage, un produit à base d'oeufs, un condiment, un mélange pour soupes, un en-cas, un produit à base de noix, un produit à base de protéines végétales, un bonbon dur, un bonbon mou, un produit à base de volaille, un jus de fruit préparé, un sucre granulé, une sauce, un jus de viande, un sirop, une barre nutritive, une boisson, une poudre déshydratée pour boissons, une confiture ou une gelée, un produit à base de poisson ou un aliment pour animaux de compagnie.

19. Denrée alimentaire suivant la revendication 17, ladite denrée alimentaire consistant en pain, tortillas, céréales, saucisse, poulet, crème glacée, yaourt, lait, assaisonnement pour salades, son de riz, jus de fruit, poudre déshydratée pour boissons, petits pains, petits gâteaux, biscuits secs, tartes aux fruits ou pâtisseries.

20. Utilisation d'une quantité efficace du composé de l'une quelconque des revendications 1 à 4, dans la production d'un médicament destiné à abaisser les taux de cholestérol total, ou les taux de glycérides, à augmenter les taux de HDL ou à avoir une association de ces effets chez un sujet.

21. Utilisation d'une quantité efficace du composé de l'une quelconque des revendications 1 à 4, dans la production d'un médicament destiné à avoir un ou plusieurs des effets suivants :
i. réduction de l'hyperglycémie chez un sujet ;
ii. réduction de l'hypercholestérolémie chez un sujet ;
iii. augmentation de la circulation chez un sujet ;
iv. stimulation du système immunitaire chez un sujet ;
v. réduction des effets secondaires de la chimiothérapie chez un sujet ;
vi. traitement ou prévention d'une maladie cardiaque dégénérative chez un sujet.

22. Utilisation d'une quantité efficace du composé de l'une quelconque des revendications 1 à 4, dans la production d'un médicament destiné au traitement de la prévention d'une affection ou maladie mitochondriale chez un sujet.

23. Utilisation suivant la revendication 22, dans laquelle l'affection est la mitochondriopathie.

24. Utilisation suivant la revendication 23, dans laquelle la mitochondriopathie est la déficience en coenzyme Q₁₀, la déficience en ubiquinone-cytochrome c-oxydoréductase, la déficience en cytochrome c-oxydase, le syndrome d'ophtalmoplégie externe progressif chronique, la dégénérescence maculaire due à l'âge, la neuropathie, l'ataxie, ou la rétinite pigmentée.

25. Formulation pharmaceutique comprenant le composé de l'une quelconque des revendications 1 à 4 et un support pharmaceutique.
